# DEMANDE DE BREVET EUROPEEN

(11) **EP 1 588 688 A1**
(43) Date de publication de la demande: **26.10.2005**
(21) Numéro de dépôt: 05300264.8
(22) Date de dépôt: 08.04.2005
(51) Int. Cl.: A61K 7/027, A61K 7/02

(54) **Gamme de compositions destinées à être appliquées sur la peau, les lèvres et/ou les phanères.**

(30) Priorité: 08.04.2004 FR 0450715
(71) Demandeur: L'OREAL, 75008 Paris (FR)
(72) Inventeur: Blin, Xavier, 75015 Paris (FR)
(74) Mandataire: Tanty, François

(57) **Abrégé**

La présente invention concerne une gamme de compositions solides de maquillage destinées à être appliquées sur la peau, les lèvres ou les phanères, comportant au moins :
- une première composition présentant une première couleur due au moins partiellement à la présence d'un premier pigment composite, et
- une deuxième composition présentant une deuxième couleur différente de la première et due au moins partiellement à la présence d'un deuxième pigment composite différent du premier.

Chaque pigment composite comporte des particules comportant :
- un noyau,
- au moins un enrobage au moins partiel d'au moins une matière colorante, les matières colorantes des premier et deuxième pigments composites étant différentes.

## Description

La présente invention concerne les compositions cosmétiques solides de maquillage destinées à être appliquées sur la peau, y compris les muqueuses, notamment les lèvres, ou les phanères, et en particulier une gamme comportant plusieurs de ces compositions.

La présente invention concerne plus particulièrement une gamme d'au moins deux compositions se présentant sous forme de stick, par exemple des rouges à lèvres.

Pour réaliser une gamme de compositions de différentes couleurs, il est connu d'incorporer dans ces compositions des pigments conférant à celles-ci des couleurs différentes. Les pigments choisis peuvent être de type organique ou inorganique et être de natures différentes dans deux compositions d'une même gamme. Les natures différentes des pigments incorporés dans des compositions sous forme de stick entraînent une variabilité des propriétés mécaniques des sticks, du fait notamment du comportement différent des pigments vis-à-vis des autres constituants de la composition, par exemple des huiles.

Il existe un besoin pour bénéficier d'une gamme de compositions présentant des couleurs différentes mais ayant des propriétés mécaniques similaires.

L'invention vise notamment à répondre à ce besoin.

Elle y parvient grâce à une gamme de compositions solides de maquillage destinées à être appliquées sur la peau, les lèvres ou les phanères, comportant au moins une première composition présentant une première couleur due au moins partiellement à la présence d'un premier pigment composite, et une deuxième composition présentant une deuxième couleur différente de la première et due au moins partiellement à la présence d'un deuxième pigment composite différent du premier, chaque pigment composite comportant des particules comportant :
- un noyau,
- au moins un enrobage au moins partiel d'au moins une matière colorante,
les matières colorantes des premier et deuxième pigments composites étant différentes.

Les première et deuxième compositions sont de préférence telles que l'écart de dureté entre elles soit inférieur ou égal à environ 25 g (0,245 N), mieux inférieur ou égal à environ 20 g (0,196 N).

En particulier, les natures des noyaux des premier et deuxième pigments composites peuvent être telles que l'écart de dureté entre les première et deuxième compositions est inférieur ou égal à environ 25 g (0,245 N), mieux inférieur ou égal à environ 20 g (0,196 N).

Grâce à l'invention, il est possible d'obtenir des compositions ayant des couleurs différentes mais des duretés relativement proches, et de constituer ainsi une gamme de compositions homogène en dureté.

Les premier et deuxième pigments composites peuvent avantageusement présenter des noyaux en un même matériau.

Une teinte appropriée de chaque composition de la gamme peut être obtenue de diverses façons, par exemple par le mélange de pigments composites selon l'invention, ces pigments ayant des couleurs différentes et/ou par la présence de plusieurs matières colorantes organiques dans l'enrobage des noyaux du ou des pigments composites, ces matières colorantes organiques étant par exemple mélangées ou présentes dans des strates respectives de l'enrobage.

Les compositions selon l'invention peuvent comporter un milieu physiologiquement acceptable.

Par « milieu physiologiquement acceptable », on désigne un milieu non toxique et susceptible d'être appliqué sur la peau, les lèvres ou les phanères d'êtres humains. Le milieu physiologiquement acceptable sera adapté à la nature du support sur lequel doit être appliquée chaque composition, ainsi qu'à la forme sous laquelle chaque composition est destinée à être conditionnée.

Par « composition cosmétique », on désigne une composition telle que définie dans la Directive 93/35/CEE du Conseil du 14 juin 1993.

La gamme peut comporter au moins trois compositions, voire au moins cinq compositions, ayant des couleurs différentes, l'écart de dureté entre elles étant inférieur ou égal à 25 g environ.

Par « écart de dureté entre elles », il faut comprendre que l'écart entre la dureté la plus faible et la dureté la plus élevée des compositions formant la gamme de produit est inférieur ou égal à 25 g environ.

Les noyaux des premier et deuxième pigments composites peuvent être inorganiques. L'enrobage de matière colorante des premier et deuxième pigments composites peut être un enrobage de matière colorante organique, ce qui permet de bénéficier d'un pouvoir colorant relativement élevé.

Les compositions de la gamme peuvent être destinées à l'application sur les lèvres et peuvent par exemple se présenter sous forme de sticks. Dans ce cas, les compositions peuvent par exemple être conditionnées dans des mécanismes comportant au moins deux parties pouvant tourner l'une par rapport à l'autre pour déplacer les sticks.

Les compositions peuvent comporter les mêmes ingrédients hormis les pigments composites. En particulier, les compositions peuvent de préférence comporter les mêmes solvants et/ou les mêmes cires.

La première et la deuxième composition peuvent être essentiellement dépourvues de matière colorante autre que les premier et deuxième pigments composites.

En variante, au moins une composition peut comporter au moins une matière colorante additionnelle différente des premier et deuxième pigments composites, la teneur en premier ou deuxième pigments composites étant par exemple supérieure ou égale à celle de ladite matière colorante additionnelle.

### Dans des exemples de mise en oeuvre de l'invention, les premier et deuxième pigments composites ne sont pas des pigments interférentiels.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite peut être supérieure ou égale à 30, mesurée selon le protocole ci-dessous.

### Protocole de mesure de la saturation du pigment composite :

Les valeurs a* et b* dans l'espace CIE L*a*b* du pigment composite sont mesurées comme suit :
Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a*² + b*²)^{1/2}.

### MESURE DE LA DURETE

Pour déterminer la dureté de la composition solide, on prépare un stick de ladite composition ayant une section circulaire de 12,7 mm de diamètre. Le stick est coulé 24 heures avant de faire la mesure et il est conservé à une température de 20 °C.

La dureté peut être mesurée par la méthode dite « du fil à couper le beurre », qui consiste à couper transversalement le stick à l'aide d'un fil rigide de diamètre 250 µm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La dureté est exprimée en grammes.

### PIGMENT COMPOSITE

### Structure

Chaque pigment composite selon l'invention peut être composé notamment de particules comportant :
- un noyau,
- au moins un enrobage au moins partiel d'au moins une matière colorante.

Au moins un liant peut avantageusement contribuer à la fixation de la matière colorante sur le noyau.

Les noyaux des premier et/ou deuxième pigments composites peuvent être inorganiques.

La matière colorante des premier et/ou deuxième pigments composites peut être organique.

La teneur en premier et/ou en deuxième pigment composite peut être comprise entre 0,1 % environ et 30 % environ en poids par rapport au poids total de la composition correspondante, voire entre 0,5 % environ et 30 % environ, par exemple entre 1 % environ et 20 % environ, voire entre environ 1 % et environ 15 %, notamment comprise entre 1 % et 10 % en poids par rapport au poids total de la composition.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

Chacune des compositions peut comporter uniquement un ou plusieurs pigments composites tels que définis plus haut ou en variante comporter un ou plusieurs pigments composites autres ainsi que des pigments présentant une structure non composite, notamment des pigments minéraux, interférentiels, des laques ou des pigments organiques. Les compositions peuvent notamment être dépourvues de particules de TiO₂ non enrobées.

### Noyau

Le noyau des premier et deuxième pigments composites, de préférence inorganique, peut être de toute forme convenant à la fixation de particules de matière colorante, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau peut présenter une taille moyenne comprise entre environ 1 nm et environ 100 nm, voire entre environ 5 nm et environ 75 nm, par exemple entre environ 10 nm et environ 50 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau peut être coloré, le cas échéant.

Le noyau inorganique peut présenter un indice de réfraction supérieur ou égal à 2, voire supérieur ou égal à 2,1, par exemple supérieur ou égal à 2,2.

La proportion massique du noyau peut excéder 50 % par rapport au poids total du pigment composite, par exemple aller de 50 % à 70 %, par exemple de 60 % à 70 %.

### Matière colorante

La matière colorante, de préférence organique, peut comporter par exemple au moins un pigment organique, par exemple au moins une laque organique.

La matière colorante peut être choisie par exemple parmi les composés particulaires insolubles dans le milieu physiologiquement acceptable de la composition.

La matière colorante peut comporter par exemple des pigments, par exemple des laques organiques ou autres matières colorantes organiques, qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La proportion massique de matière colorante organique des premier et/ou deuxième pigments composites peut être comprise entre environ 10 parts et environ 500 parts en poids pour 100 parts du noyau, voire entre environ 20 parts et environ 250 parts en poids, par exemple entre environ 40 parts et environ 125 parts en poids pour 100 parts du noyau.

La proportion de la matière colorante organique peut excéder 30 % par rapport au poids total du pigment composite, par exemple aller de 30 à 50 %, par exemple de 30 à 40%.

### Liant

Le liant peut être de tout type dès lors qu'il permet à la matière colorante organique d'adhérer à la surface du noyau. Le liant peut être organique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
   - les polysiloxanes modifiés (2A) comprenant au moins un radical choisi parmi, notamment, les polyéthers, les polyesters et les composés époxy (ils seront appelés « polysiloxanes modifiés »),
   - les polysiloxanes (2B) portant sur un atome de silicium situé à l'extrémité du polymère, au moins un groupe choisi parmi une liste non limitative comprenant les acides carboxyliques, les alcools ou les groupes hydroxy, et
   - les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ ₐ Si X₄₋ₐ (I)

dans laquelle :
- R¹ représente C₆H₅-, (CH₃)₂ CH CH₂- ou n- C_{b} H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthyoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriethyoxysilane.

Les polysiloxanes (2) peuvent notamment répondre à la formule (II) : 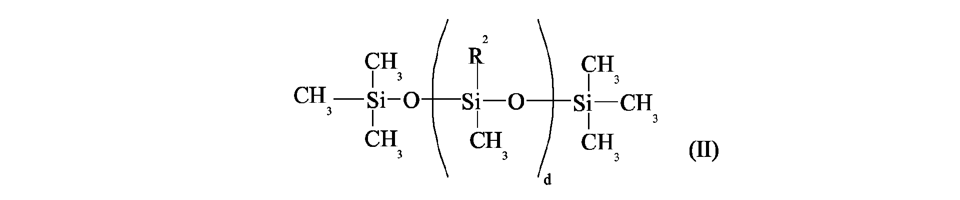 dans laquelle R² représente H- ou CH₃- et d varie de 15 à 450.

Parmi ces polysiloxanes, ceux pour lesquels R² représente H sont préférés.

Les polysiloxanes modifiés (2A) peuvent notamment répondre aux formules suivantes :
- (a¹) polysiloxanes modifiés portant des polyéthers, représentés par la formule (III) 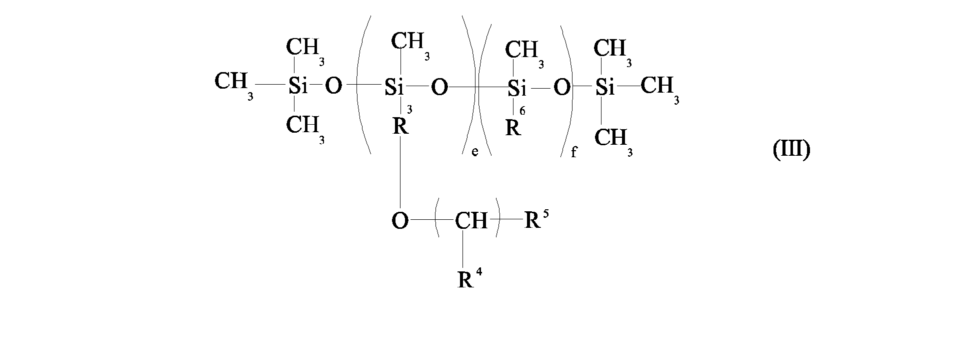 dans laquelle R³ représente -(CH₂)ₕ- ; R⁴ représente -(CH₂)ᵢ- CH₃ ; R⁵ représente -OH, - COOH, -CH = CH₂, -C (CH₃) = CH₂ ou -(CH₂)ⱼ- CH₃ ; R⁶ représente -(CH2)ₖ- CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 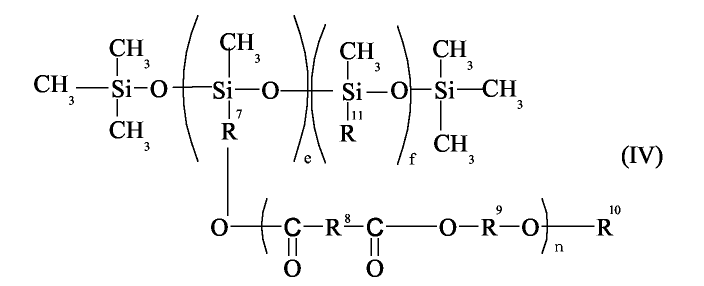 dans laquelle R⁷, R⁸ et R⁹ représentent indépendamment -(CH₂)_{q}- ; R¹⁰ représente -OH ; -COOH, -CH = CH₂, -C(CH₃) = CH₂ ou -(CH₂)ᵣ- CH₃ ; R¹¹ représente -(CH₂)ₛ- CH₃ ; n et q variant indépendamment de 1 à 15, r et s variant indépendamment de 0 à 15 ; e variant de 1 à 50 et f variant de 1 à 300,
- (a³) polysiloxanes modifiés portant des radicaux époxy représentés par la formule (V): 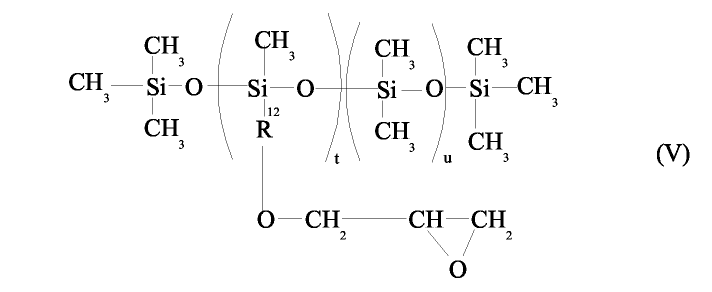
dans laquelle R¹² représente -(CH₂)ᵥ- ; v variant de 1 à 15 ; t variant de 1 à 50 et u variant de 1 à 300 ; ou leurs mélanges.
Parmi les polysiloxanes modifiés (2A), les polysiloxanes modifiés portant des polyéthers de formule (III) sont préférés.
Les polysiloxanes modifiés sur la partie terminale (2B) peuvent répondre à la formule (VI) : 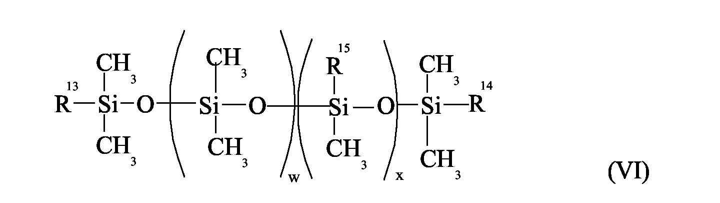 dans laquelle R¹³ et R¹⁴ peuvent représenter -OH, R¹⁶ OH ou R¹⁷ COOH, indépendamment l'un de l'autre ; R¹⁵ représente -CH₃ ou -C₆H₅ ; R¹⁶ et R¹⁷ représentent -(CH₂)_{y}- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂ (R¹⁸)ₐ SiX₄₋ₐ (VII)

dans laquelle :
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkyles silanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyl triéthoxysilane, le tridécafluorooctyl triéthoxysilane, l'heptadécafluorodecyl triéthoxysilane, l'heptadécafluorodécylméthyl diéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyl triméthoxysilane.

Les agents couplants à base de silane peuvent être choisis parmi une liste non limitative comprenant notamment le vinyltriméthoxysilane, vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)- γ-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropyl stéaroyl, le titanate d'isopropyltris(dioctylpyrophosphate), le titanate d'isopropyltri(N-aminoéthylaminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triséthylacétoacétate d'aluminium, le trisacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2. La quantité de liant est par exemple inférieure ou égale à 5 %, par exemple inférieure ou égale à 3 %, en poids relativement au poids total du pigment composite.

### Préparation des premier et deuxième pigments composites

Le pigment composite peut être préparé par tout procédé approprié, par exemple un procédé méchano-chimique ou un précédé de précipitation en solution, avec dissolution de la matière colorante organique puis précipitation à la surface du noyau.

Un liant peut être utilisé ou non.

Un procédé comportant un mélange mécanique d'un pigment organique et du noyau inorganique est préféré.

Un liant peut être ajouté et mélangé au noyau inorganique avant l'introduction de la matière colorante organique.

Chacun des premier et deuxième pigments composites peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau avec le liant.

De manière à ce que le liant adhère uniformément à la surface du noyau, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau, la matière colorante est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux avec le liant ou de la matière colorante avec les noyaux recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante est dissoute dans l'éthanol, les noyaux sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

### AUTRES COMPOSANTS

### Huile

Chaque composition selon l'invention contient avantageusement au moins une huile. L'huile peut être choisie parmi les huiles volatiles, les huiles non volatiles et leurs mélanges.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

Par « huile non volatile », on entend une huile restant sur la peau à température ambiante et pression atmosphérique au moins plusieurs heures et ayant notamment une pression de vapeur inférieure à 0,13 Pa (0,001 mm de Hg).

Ces huiles volatiles ou non volatiles peuvent être des huiles hydrocarbonées notamment d'origine animale ou végétale, des huiles siliconées, ou leurs mélanges. On entend par « huile hydrocarbonée », une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité inférieure ou égale à 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans l'une au moins des compositions de la gamme selon l'invention à une teneur allant de 0,1 % à 98 % en poids, notamment de 1% à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans l'une au moins des compositions de la gamme selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

Les huiles non volatiles peuvent être présentes dans l'une au moins des compositions selon l'invention en une teneur allant de 0,01 à 98 % en poids, notamment de 0,1 % à 85 % en poids, et en particulier de 1 % à 70 % en poids, par rapport au poids total de la composition.

Les huiles peuvent représenter de 0,01 à 99 % du poids total de la composition, de préférence de 0,05 à 60 % et mieux de 1 à 75 %.

### Cire

Au moins une des compositions selon l'invention peut comprendre au moins une cire. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 200 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 30 °C et mieux supérieure à 45 °C.

Comme cire utilisable dans au moins une composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

A titre indicatif, au moins une composition peut contenir de 1 à 50 % en poids de cires, par rapport au poids total de la composition et mieux de 5 à 30 % en poids.

### Charges

Au moins une composition selon l'invention peut comprendre en outre une ou plusieurs charges, notamment en une teneur allant de 0,01 % à 50 % en poids, par rapport au poids total de la composition, de préférence allant de 0,01 % à 30 % en poids, et préférentiellement allant de 0,01 % à 10 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez ATOCHEM), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (NOBEL INDUSTRIE), de copolymères d'acide acrylique (Polytrap® de la société DOW CORNING) et les microbilles de résine de silicone (Tospearls® de TOSHIBA, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Matière colorante additionnelle

Au moins l'une des compositions de la gamme peut comprendre au moins une matière colorante additionnelle, différente des premier et deuxième pigments composites utilisés dans la présente invention.

La matière colorante additionnelle peut être choisie parmi les pigments minéraux, les pigments organiques, les pigments nacrés, les colorants liposolubles ou hydrosolubles.

Les pigments minéraux peuvent être blancs ou colorés, enrobés ou non. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

Parmi les pigments organiques, on peut citer le noir de carbone, les pigments de type D & C, et les laques à base de carmin de cochenille, de baryum, strontium, calcium, aluminium.

Les colorants liposolubles sont par exemple le rouge Soudan, le D&C Red N° 17, le D1C Green N° 6, le β-carotène, l'huile de soja, le brun Soudan, le D&C Yellow N° 11, le D&C Violet N° 2, le D&C orange N° 5, le jaune quinoléine.

Les colorants hydrosolubles sont par exemple le jus de betterave et le bleu de méthylène.

Les colorants peuvent représenter de 0,1 à 20 % du poids de la composition et mieux de 0,1 à 6 % (si présents).

### Autres ingrédients

Avantageusement, au moins une des compositions, voire chacune des compositions de la gamme, peut être une composition anhydre, c'est-à-dire une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Au moins une composition peut contenir d'autres ingrédients cosmétiques usuels pouvant être choisis notamment parmi les antioxydants, les polymères filmogènes, les parfums, les conservateurs, les épaississants comme le bentone, les neutralisants, les tensioactifs, les filtres solaires, les vitamines, les hydratants, les actifs cosmétiques ou dermatologiques.

Bien entendu, l'homme du métier veillera à choisir ce ou ces éventuels composés complémentaires, et/ou leur quantité, de manière telle que les propriétés avantageuses de cette composition selon l'invention ne soient pas, ou substantiellement pas, altérées par l'adjonction envisagée.

Les compositions solides de maquillage selon l'invention peuvent être des compositions de maquillage de la peau ou des lèvres, telles que des fonds de teint, des fards à paupières, des fards à joue, des produits anticernes, des produits de maquillage du corps, des rouges à lèvres. Plus spécialement mais non exclusivement, l'invention porte sur des compositions de rouges à lèvres.

### EXEMPLES

On a réalisé plusieurs rouges à lèvres sous forme de sticks ayant la composition suivante (quantités exprimées en % massique par rapport au poids total de la composition) :

| | |
|---|---|
| Octyl-2 dodécanol | 15,63 |
| Ditertiobutyl 4-hydroxytoluène | 0,06 |
| Lanolate d'isopropyle | 9,59 |
| Triglycérides d'acides laurique/palmitique/catylique/stéarique -50/20/10/10 (Softisan 100 de la société SASOL) | 5 |
| Lanoline acétylée protégée | 9,59 |
| Phényl triméthylsiloxy trisiloxane | 4,26 |
| Cire de polyéthylène (PM : 500) (Polywax 500 de la société | 8,8 |
| BARECO) | |
| Malate de di-iso-stréaryle | 13,07 |
| Lanoline liquide | 9,60 |
| Tri-méllitate de tri-décyle | 10,40 |
| Cire microcristalline | 4 |
| Matière colorante testée | 10 |

Toutes les compositions testées comportent la formulation de base ci-dessus et diffèrent par la nature de la matière colorante, présente dans une proportion massique de 10%.

### Exemples 1, 2 et 3 conformes à l'invention

L'exemple 1 de rouge à lèvres comporte un pigment composite selon l'invention.

La matière colorante utilisée dans l'exemple 1 est un pigment composite constitué de 50 parts en poids de pigment organique de dénomination D&C Red N° 7 pour 100 parts d'un noyau inorganique de dioxyde de titane de taille moyenne 20 nm et de surface spécifique 50 m²/g, et réalisé avec 1,3 parts d'un liant polyméthylhydrogénosiloxane.

Le rouge à lèvres de l'exemple 2 comporte un pigment composite selon l'invention constitué de 50 parts en poids de pigment organique de dénomination FD&C Yellow N° 5 pour 100 parts d'un noyau inorganique de dioxyde de titane de taille moyenne 20 nm et de surface spécifique 50 m²/g, et réalisé avec 1,3 parts d'un liant polyméthylhydrogénosiloxane.

Le rouge à lèvres de l'exemple 3 conforme à l'invention comporte un pigment composite comportant 70 parts en poids de laque organique de dénomination FD&C Blue 1 AL lake pour 100 parts d'un noyau inorganique de dioxyde de titane de taille moyenne 20 nm et de surface spécifique 50 m²/g, et réalisé avec un liant polyméthylhydrogénosiloxane.

Les exemples 1, 2 et 3 constituent une gamme de rouges à lèvres de couleurs différentes. On a mesuré la dureté des sticks obtenus avec ces trois compositions à l'aide de la méthode décrite plus haut.

La dureté mesurée pour le stick de l'exemple 1 est de 149 g (soit 1,46 N) environ.

La dureté mesurée pour le stick de l'exemple 2 est de 167 g (soit 1,636 N) environ.

La dureté mesurée pour le stick de l'exemple 3 est de 163 g (soit 1,597 N) environ.

Ainsi, l'écart maximum de dureté pour ces trois compositions d'une même gamme est de l'ordre de 18 g (soit 0,176 N).

### Exemples comparatifs 1, 2 et 3 :

On a réalisé trois compositions comportant la formulation de base précédemment décrite et incorporant comme matière colorante pour l'exemple comparatif 1, un pigment organique D&C Red N° 7, pour l'exemple comparatif 2 un pigment organique FD&C Yellow N° 5, et pour l'exemple comparatif 3 une laque organique FD&C Blue 1 A1 lake, les trois matières colorantes étant en proportion massique de 10 % dans la composition.

Pour l'exemple comparatif 1, la dureté mesurée est de 117 g (soit 1,146 N) environ.

Pour l'exemple comparatif 2, la dureté mesurée est de 166 g (soit 1,626 N) environ.

Pour l'exemple comparatif 3, la dureté mesurée est de 155 g (soit 1,519 N) environ.

Ainsi, on obtient un écart de dureté relativement important de l'ordre de 49 g (soit 0,48 N), pour cette gamme de sticks composée des exemples comparatifs 1, 2 et 3.

### Exemples comparatifs 4, 5 et 6 :

On a réalisé trois autres compositions sous forme de sticks de rouges à lèvres incorporant une proportion de 10 % en poids des matières colorantes suivantes.

L'exemple comparatif 4 comporte un mélange de nano TiO₂ (6,21 %) et de pigment organique D&C Red N° 7 (3,29 %).

L'exemple comparatif 5 comporte un mélange de nanotitane (6,21 %) et de pigment organique FD&C Yellow N° 5 (3,29 %).

L'exemple comparatif 6 comporte un mélange de nanotitane (5,93 %) et de laque organique FD&C Blue 1 A1 lake (4,07 %).

Les duretés mesurées sont les suivantes.

Pour l'exemple comparatif 4, la dureté mesurée est de 106 g (soit 1,039 N) environ.

Pour l'exemple comparatif 5, la dureté mesurée est de 141 g (soit 1,382 N) environ.

Pour l'exemple comparatif 6, la dureté mesurée est de 143 g environ (soit 1,401 N).

Ainsi, l'écart de dureté entre ces trois compositions d'une même gamme de sticks est d'environ 37 g (soit 0,36 N).

Les essais réalisés mettent en évidence que les compositions (exemples 1, 2 et 3) de la gamme selon l'invention à base de pigments composites présentent un écart de dureté inférieur à 20g (soit 0,196 N) , malgré les natures différentes des matières colorantes des pigments composites.

Bien entendu, l'invention n'est pas limitée aux exemples qui viennent d'être donnés.

Dans toute la description, y compris les revendications, l'expression « comportant un » doit être comprise comme étant synonyme de « comportant au moins un », sauf si le contraire est spécifié.

Les intervalles donnés doivent être compris bornes incluses, sauf si le contraire est spécifié.

## Revendications

1. Gamme de compositions solides de maquillage destinées à être appliquées sur la peau, les lèvres ou les phanères, comportant au moins :
- une première composition présentant une première couleur due au moins partiellement à la présence d'un premier pigment composite, et
- une deuxième composition présentant une deuxième couleur différente de la première et due au moins partiellement à la présence d'un deuxième pigment composite différent du premier,
chaque pigment composite comportant des particules comportant :
- un noyau,
- au moins un enrobage au moins partiel d'au moins une matière colorante,
les matières colorantes des premier et deuxième pigments composites étant différentes, l'écart de dureté entre les première et deuxième compositions étant de préférence inférieur ou égal à environ 25 g (0,245 N).

2. Gamme selon la revendication 1, **caractérisée par le fait que** les natures des noyaux des premier et deuxième pigments composites sont telles que l'écart de dureté entre les première et deuxième compositions est inférieur ou égal à environ 25 g (0,245 N).

3. Gamme selon l'une des revendications 1 et 2, **caractérisée par le fait que** les premier et deuxième pigments composites présentent des noyaux en un même matériau.

4. Gamme selon l'une quelconque des revendications précédentes, **caractérisée par le fait qu'**elle comporte au moins trois compositions de couleurs différentes ayant un écart de dureté entre elles inférieur ou égal à 25 g (0,245 N) environ.

5. Gamme selon l'une quelconque des revendications précédentes, **caractérisée par le fait qu'**elle comporte au moins cinq compositions de couleurs différentes ayant un écart de dureté entre elles inférieur à 25 g (0,245 N) environ.

6. Gamme selon l'une quelconque des revendications précédentes, **caractérisée par le fait que** les noyaux des premier et deuxième pigments composites sont inorganiques.

7. Gamme selon l'une quelconque des revendications précédentes, **caractérisée par le fait que** l'enrobage de matière colorante des premier et deuxième pigments composites est un enrobage de matière colorante organique.

8. Gamme selon l'une quelconque des revendications précédentes, **caractérisée par le fait que** les compositions sont destinées à l'application sur les lèvres.

9. Gamme selon l'une quelconque des revendications précédentes, **caractérisée par le fait que** les compositions se présentent sous forme de sticks.

10. Gamme selon la revendication 9, **caractérisée par le fait que** les compositions sont conditionnées dans des mécanismes comportant au moins deux parties pouvant tourner l'une par rapport à l'autre pour déplacer les sticks.

11. Gamme selon l'une quelconque des revendications précédentes, **caractérisée par le fait que** les compositions comportent les mêmes ingrédients hormis les pigments composites.

12. Gamme selon l'une quelconque des revendications précédentes, **caractérisée par le fait que** les compositions comportent les mêmes solvants.

13. Gamme selon l'une quelconque des revendications précédentes, **caractérisée par le fait que** les compositions comportent les mêmes cires.

14. Gamme selon l'une quelconque des revendications précédentes, **caractérisée par le fait que** l'écart de dureté entre la première et la deuxième composition est inférieur ou égal à environ 20 g (0,196 N).

15. Gamme selon l'une quelconque des revendications précédentes, **caractérisée par le fait que** la première et la deuxième composition sont essentiellement dépourvues de matière colorante autre que les premier et deuxième pigments composites.

16. Gamme selon l'une quelconque des revendications 1 à 14, **caractérisée par le fait qu'**au moins une composition comporte au moins une matière colorante additionnelle différente des premier et deuxième pigments composites, la teneur en premier ou deuxième pigment composite étant supérieure ou égale à celle de ladite matière colorante additionnelle.

17. Gamme selon l'une quelconque des revendications précédentes, **caractérisée par le fait que** la teneur en premier et/ou en deuxième pigment composite est comprise entre environ 0,1 % et environ 30 % en poids, par rapport au poids total de la composition.

18. Gamme selon la revendication précédente, **caractérisée par le fait que** la teneur en premier et/ou en deuxième pigment composite est comprise entre environ 0,5 % et environ 30 % en poids par rapport au poids total de la composition.

19. Gamme selon la revendication précédente, **caractérisée par le fait que** la teneur en premier et/ou en deuxième pigment composite dans la composition est comprise entre environ 1 % et environ 20 % en poids par rapport au poids total de la composition, mieux entre environ 1 % et environ 15 % en poids, notamment comprise entre 1 % et 10%.

20. Gamme selon l'une quelconque des revendications précédentes, **caractérisée par le fait que** les noyaux des premier et/ou deuxième pigments composites présentent chacun une taille moyenne comprise entre environ 1 nm et environ 100 nm.

21. Gamme selon l'une quelconque des revendications précédentes, **caractérisée par le fait que** les noyaux des premier et/ou deuxième pigments composites présentent une taille moyenne comprise entre environ 5 nm et environ 75 nm.

22. Gamme selon la revendication précédente, **caractérisée par le fait que** les noyaux des premier et/ou deuxième pigments composites présentent une taille moyenne comprise entre environ 10 nm et environ 50 nm.

23. Gamme selon l'une quelconque des revendications précédentes, **caractérisée par le fait que** les noyaux des premier et/ou deuxième pigments composites présentent une surface spécifique comprise entre environ 1 m²/g et environ 1000 m²/g.

24. Gamme selon la revendication précédente, **caractérisée par le fait que** la surface spécifique des noyaux est comprise entre environ 10 m²/g et environ 600 m²/g.

25. Gamme selon la revendication précédente, **caractérisée par le fait que** la surface spécifique des noyaux est comprise entre environ 20 m²/g et environ 400 m²/g.

26. Gamme selon l'une quelconque des revendications précédentes, **caractérisée par le fait que** les noyaux des premier et/ou deuxième pigments composites présentent une forme choisie parmi les suivantes : sphérique, globulaire, polyédrique, aciculaire, fusiforme, aplatie.

27. Gamme selon l'une quelconque des revendications précédentes, **caractérisée par le fait que** les noyaux des premier et/ou deuxième pigments composites comportent un matériau choisi parmi un sel métallique, un oxyde métallique, une alumine, un verre, une céramique, un graphite, une silice, un silicate, un mica synthétique, et un de leurs mélanges.

28. Gamme selon la revendication précédente, **caractérisée par le fait que** les noyaux comportent un oxyde métallique choisi parmi un oxyde de titane, de zirconium, de cérium, de zinc, de fer, de bleu ferrique, de chrome et d'aluminium.

29. Gamme selon la revendication précédente, **caractérisée par le fait que** l'oxyde métallique est choisi parmi un oxyde de titane, de fer, de cérium, de zirconium, de zinc et d'aluminium.

30. Gamme selon la revendication précédente, **caractérisée par le fait que** les noyaux comportent du dioxyde de titane.

31. Gamme selon la revendication 27, **caractérisée par le fait que** les noyaux comportent au moins un silicate choisi parmi un aluminosilicate et un borosilicate.

32. Gamme selon la revendication 27, **caractérisée par le fait que** les noyaux comportent une silice.

33. Gamme selon l'une quelconque des revendications précédentes, **caractérisée par le fait que** la proportion massique de matière colorante des premier et/ou deuxième pigments composites est comprise entre environ 10 et environ 500 parts en poids pour 100 parts en poids du noyau.

34. Gamme selon la revendication précédente, **caractérisée par le fait que** la proportion massique de matière colorante des premier et/ou deuxième pigments composites est comprise entre environ 20 et environ 250 parts en poids pour 100 parts en poids du noyau.

35. Gamme selon la revendication précédente, **caractérisée par le fait que** la proportion massique de matière colorante des premier et/ou des deuxième pigments composites est comprise entre environ 40 et environ 125 parts en poids pour 100 parts en poids du noyau.

36. Gamme selon l'une quelconque des revendications précédentes, **caractérisée par le fait que** la matière colorante des premier et/ou deuxième pigments composites comporte au moins un pigment organique.

37. Gamme selon l'une quelconque des revendications précédentes, **caractérisée par le fait que** la matière colorante des premier et/ou deuxième pigments composites comporte au moins une laque organique.

38. Gamme selon l'une quelconque des revendications précédentes, **caractérisée par le fait que** la matière colorante des premier et/ou deuxième pigments composites est choisi parmi un carmin de cochenille, un pigment organique de colorant azoïque, anthraquinonique, indigoïde, xanthénique, pyrénique, quinolinique, de triphénylméthane, de fluorane, une laque organique, un sel insoluble de sodium, de potassium, de calcium, de baryum, d'aluminium, de zirconium, de strontium, de titane, de colorants acides.

39. Gamme selon la revendication précédente, **caractérisée par le fait que** le colorant acide est choisi parmi un colorant azoïque, anthraquinonique, indigoïde, xanthénique, pyrénique, quinolinique, de triphénylméthane, de fluorane, et tout autre colorant comportant au moins un groupe acide carboxylique ou sulfonique.

40. Gamme selon l'une quelconque des revendications précédentes, **caractérisée par le fait que** la matière colorante des premier et/ou deuxième pigments composites est choisie parmi les pigments organiques suivants : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

41. Gamme selon l'une quelconque des revendications précédentes, **caractérisée par le fait que** la matière colorante des premier et/ou deuxième pigments composites est choisies parmi les laques organiques suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

42. Gamme selon l'une quelconque des revendications précédentes, **caractérisée par le fait que** la matière colorante des premier et/ou deuxième pigments composites comporte une laque organique supportée par un support organique comportant au moins une colophane ou du benzoate d'aluminium.

43. Gamme selon l'une quelconque des revendications précédentes, **caractérisée par le fait que** les premier et/ou deuxième pigments composites comportent au moins un liant contribuant à la fixation de la matière colorante sur le noyau.

44. Gamme selon la revendication précédente, **caractérisée par le fait que** le liant comporte au moins l'un d'un composé siliconé, d'un composé polymérique, d'un composé oligomérique et similaire comportant au moins l'un d'un organe de silane, d'un organosilanefluoroalkylé, d'un polysiloxane, d'un agent couplant et d'un mélange de ceux-ci.

45. Gamme selon la revendication précédente, **caractérisée par le fait que** l'agent couplant est à base de silane, de titanate, d'aluminate et/ou de zirconate, ou d'un mélange de ceux-ci.

46. Gamme selon la revendication 44, **caractérisée par le fait que** le liant comporte au moins un composé siliconé.

47. Gamme selon l'une quelconque des revendications précédentes, **caractérisée par le fait que** les noyaux des premier et/ou deuxième pigments composites sont colorés.

48. Gamme selon l'une quelconque des revendications précédentes, **caractérisée par le fait que** les première et deuxième compositions sont dépourvues de particules de dioxyde de titane non enrobées.

49. Gamme selon l'une quelconque des revendications précédentes, **caractérisée par le fait que** les première et deuxième compositions comportent au moins un actif cosmétique ou dermatologique.

50. Gamme selon l'une quelconque des revendications précédentes, **caractérisée par le fait que** les première et deuxième compositions comportent au moins une huile ou une cire.

51. Gamme selon la revendication 16, **caractérisée par le fait que** la matière colorante additionnelle comporte au moins l'un d'un pigment minéral, d'un pigment organique, d'un pigment nacré et d'un colorant liposoluble ou hydrosoluble.

52. Gamme selon la revendication 51, **caractérisée par le fait que** la matière colorante additionnelle comporte un pigment minéral comportant du dioxyde de titane, un oxyde de zirconium, de cérium, de fer, de chrome, le violet de manganèse, le bleu outremer, l'hydrate de chrome ou le bleu ferrique.

53. Gamme selon la revendication 51, **caractérisée par le fait que** la matière colorante additionnelle comporte un pigment organique comportant du noir de carbone, un pigment de type D&C ou une laque à base de carmin de cochenille, de baryum, de strontium, de calcium ou d'aluminium.

54. Gamme selon la revendication 51, **caractérisée par le fait que** la matière colorante additionnelle comporte un pigment nacré blanc ou coloré.

55. Gamme selon la revendication 51, **caractérisée par le fait que** la matière colorante additionnelle comporte un colorant liposoluble comportant un rouge Soudan, un D&C Red N° 17, un D&C Green N° 6, un β-carotène, une huile de jojoba, un brun Soudan, un D&C Yellow N° 11, un D&C Violet N° 2, un D&C orange N° 5 et/ou un jaune quinoléine.

56. Gamme selon la revendication 43, **caractérisée par le fait que** le liant est organique.

57. Gamme selon l'une quelconque des revendications précédentes, **caractérisée par le fait que** la matière colorante organique est différente de la mélanine.

58. Gamme selon l'une quelconque des revendications précédentes, **caractérisée par le fait que** les premier et deuxième pigments composites ont une saturation C* supérieure ou égale à 30.

59. Gamme selon l'une quelconque des revendications précédentes, **caractérisée par le fait que** les premier et deuxième pigments composites sont des pigments non interférentiels.

60. Gamme selon la revendication 43, **caractérisée par le fait que** la quantité relative de liant est inférieure ou égale à 5% par rapport au poids total du pigment composite.

61. Gamme selon l'une quelconque des revendications précédentes, exceptée la revendication 8, **caractérisée par le fait que** les compositions sont destinées au maquillage de la peau.

62. Gamme selon la revendication précédente, **caractérisée par le fait que** les compositions sont des fonds de teint.

63. Gamme selon la revendication 61, **caractérisée par le fait que** les compositions sont des fards à paupières.

64. Gamme selon la revendication 61, **caractérisée par le fait que** les compositions sont des fards à joue.

65. Gamme selon la revendication 61, **caractérisée par le fait que** les compositions sont des produits anticernes.

66. Gamme selon la revendication 61, **caractérisée par le fait que** les compositions sont des produits de maquillage du corps.

67. Gamme selon la revendication 8, **caractérisée par le fait que** les compositions sont des rouges à lèvres.
